# EUROPEAN PATENT APPLICATION

(11) **EP 3 400 903 A1**
(43) Date of publication of application: **14.11.2018**
(21) Application number: 18275064.6
(22) Date of filing: 09.05.2018
(51) Int. Cl.: A61F 2/07

(54) **SELF-ADAPTING GRAFT FOR PATIENTS**

(30) Priority: 10.05.2017 US 201762504212 P
(71) Applicant: Cook Medical Technologies LLC, Bloomington, IN 47404 (US)
(72) Inventor: Sumanasinghe, Ruwan, Carmel, IN 46032 (US); Spindler, Ralf, Solsberry, IN 47459 (US)
(74) Representative: Williams Powell

(57) **Abstract**

In one example, a prosthesis comprises a generally tubular graft material (14) having an inner and outer perimeter, and a plurality of pleats (16) between the inner and outer perimeters in a first state. At least one stent (12) is coupled to the graft material. At least one layer of biocompatible material is coupled to the graft material in the first state. One or more sections of the at least one layer biodegrade over a first period of time at a first biodegradable rate. At least one of the plurality of pleats (16) of the graft material unfolds to permit radial expansion of the graft material after the first period of time, and the stent provides support for the radial expansion of the graft material.

## Description

### TECHNICAL FIELD

This present disclosure relates to medical devices, and more particularly to stent-grafts that are self-adapting to account for anatomy changes, such as anatomy changes in younger patients.

### BACKGROUND

Stent-grafts are commonly used in the medical field to treat aneurysms and many other diseases. In one example, endovascular techniques have been developed with the use of stent-graft deployments as a newer and less invasive form than open surgery for the treatment of abdominal aortic aneurysms (AAA).

During stent-graft therapy, small incisions may be made at one or more locations for entry of a delivery device. Using imaging techniques, a surgeon may deliver the device to the target site, in this example an aneurysm site in the abdomen. Such delivery is less invasive and may be preferred over other and more intrusive forms of surgery. The stent-graft is then released from the delivery device into the aorta. As the stent-graft is released, it expands in size so that it fits into the aorta, preferably at locations that engage a vessel wall proximal and distal to the aneurysm. The delivery device is then withdrawn and removed, leaving the stent-graft within the aorta. Depending on the shape and size of the aortic aneurysm, additional stent-grafts may be placed to ensure that the aneurysm is sufficiently excluded from blood flow.

One common practice is to expand or enlarge a stent-graft in pediatric patients to accommodate the growth of the vessel. Typically, a balloon catheter is used to periodically expand the stent-graft. However, this expansion is limited by the diameter of the tubular graft, whereby after a certain maximum diameter, the stent and the graft cannot be further expanded.

### SUMMARY OF INVENTION

Aspects of the present invention seek to provide an improved prosthesis.

According to an aspect of the invention, there is provided a prosthesis as in claim 1.

According to an aspect of the invention, there is provided a prosthesis as in claim 11.

In one example, a prosthesis comprises a generally tubular graft material having an inner and outer perimeter, and a plurality of pleats between the inner and outer perimeters in a first state. At least one stent is coupled to the graft material. At least one layer of biocompatible material is coupled to the graft material in the first state. One or more sections of the at least one layer biodegrade over a first period of time at a first biodegradable rate. At least one of the plurality of pleats of the graft material unfolds to permit radial expansion of the graft material after the first period of time, and the stent provides support for the radial expansion of the graft material.

Embodiments provide stent-graft designs to accommodate the periodic expansion of a vessel due to its growth that reduces or eliminates further interventional expansion procedures. Embodiments may adapt to changes in vessel diameters of the patients, thereby reducing the need to continuously monitor and further expand stent-grafts.

Other systems, methods, features and advantages of the embodiments of invention will be, or will become, apparent to one with skill in the art upon examination of the following figures and detailed description. It is intended that all such additional systems, methods, features and advantages be within the scope of the invention, and be encompassed by the following claims.

### BRIEF DESCRIPTION OF DRAWINGS

Embodiments of the invention can be better understood with reference to the following drawings and description, which are provided by way of example only. The components in the figures are not necessarily to scale, emphasis instead being placed upon illustrating the principles of embodiments of the invention. Moreover, in the figures, like referenced numerals designate corresponding parts throughout the different views.
FIGS. 1A-1B are side and perspective views of examples of a folded graft and at least one stent for use in a stent-graft according to a first embodiment.
FIG. 2 shows a partial perspective view of the folded graft of FIG. 1 placed in between biodegradable inner and outer layers.
FIG. 3 shows a segment of a graft with tissue growth inhibitors embedded within the spaces between the inner and outer layers.
FIGS. 4A-4D show exemplary stages of expansion of the stent-graft of FIGS. 1-3.
FIG. 5 is a perspective view of another embodiment of a stent-graft.
FIG. 6 is a perspective view of another embodiment of a stent-graft.
FIGS. 7A-7C show embodiments of a segment of a graft disposed adjacent to one or more biodegradable layers.
FIGS. 8A-8C show embodiments a segment of a graft disposed adjacent to a biodegradable layer.
FIGS. 9A-9C show further embodiments a segment of a graft disposed adjacent to a biodegradable layer.

### DETAILED DESCRIPTION

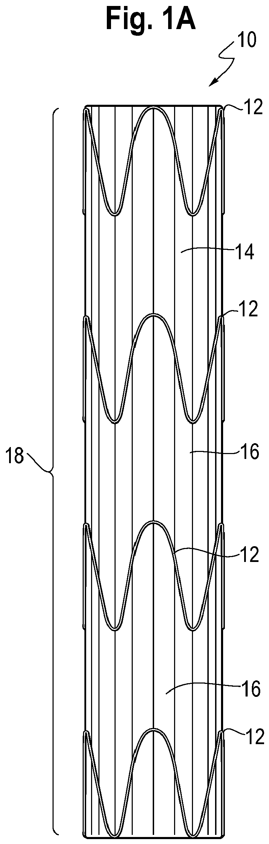

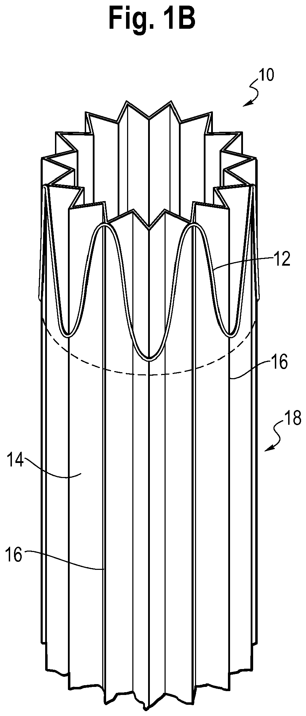

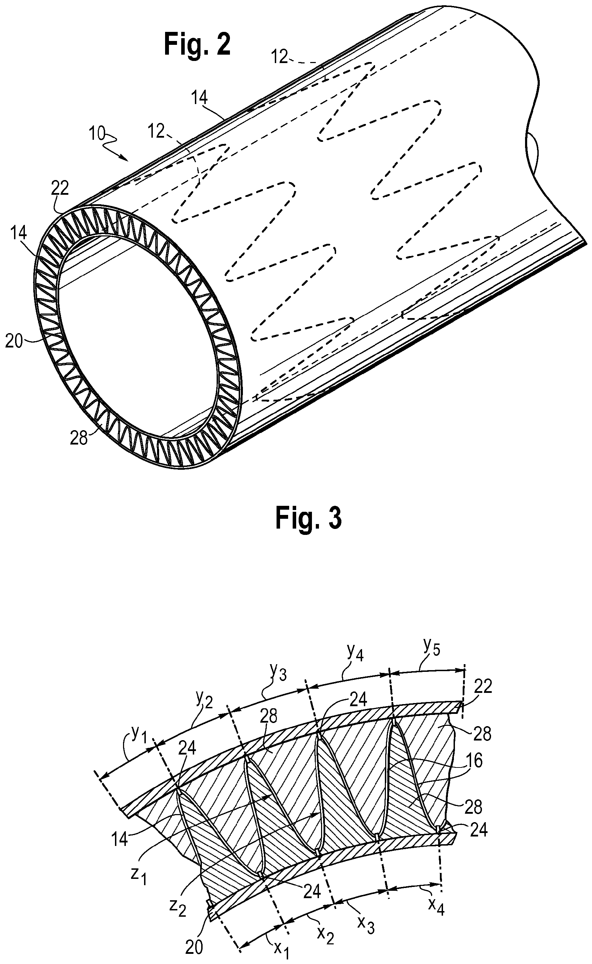

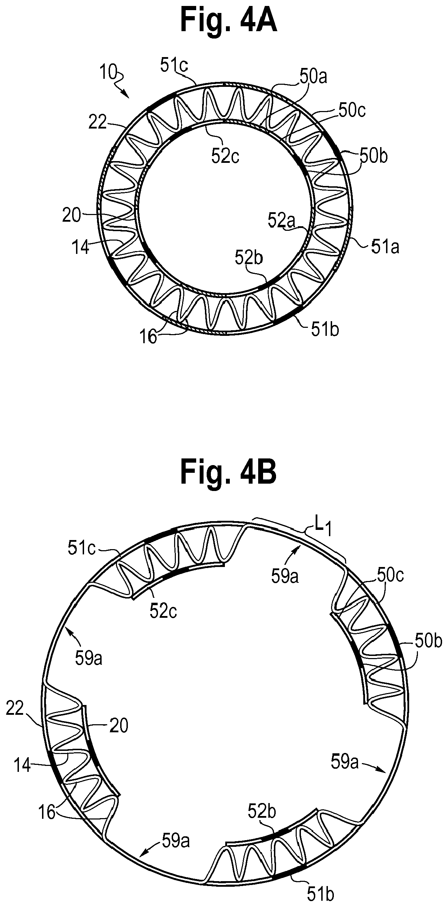

Referring to FIGS. 1-4, a first embodiment of a stent-graft 10 is shown and described. It should be noted that in FIGS. 1A-1B, a tubular graft material 14 and at least one stent 12 are shown in an isolated manner, with inner and outer biodegradable layers 20 and 22 of FIGS. 2-4 being omitted for illustrative purposes. It is also noted that in FIG. 3 and FIGS. 4A-4C, while the inner and outer biodegradable layers 20 and 22 are depicted, the one or more stents 12 of FIGS. 1A-1B are omitted for illustrative purposes.

Referring to FIGS. 1A-1B, the stent-graft 10 comprises the tubular graft material 14 having a body region 18 and a plurality of pleats 16, with the at least one stent 12 supporting the body region 18. The tubular graft material 14 has an inner and outer perimeter, and the plurality of pleats 16 are disposed between the inner and outer perimeters in a first state, as best seen in FIGS. 1B, 2 and 4A.

In this non-limiting example, the pleats 16 are shown as uninterrupted longitudinal pleats in a radial, accordion pleating pattern, in which they extend between the inner and outer perimeters of the graft material 14. The pleats 16 are shown as uniform, but may be non-uniform about the circumference of the stent-graft 10. In further examples, the pleats 16 may reside on part of the body region 18 between its proximal and distal ends, or about its circumference, while other parts of the body region 18 may omit the pleats 16 in the state of FIGS. 1A-1B.

In one example, the one or more stents 12 may be secured at every peak of the pleats 16. In other embodiments, the one or more stents 12 may be secured to every other peak of the pleats 16, or to a variable number of pleats 16 depending on needs of a particular patient and procedure.

The stent-graft 10 may be deployed into a patient through common delivery methods to a vessel in the body. As the body vessel grows or changes in diameter, the pleats 16 allow the graft material 14 to radially expand further, as the stent 12 is allowed to expand as explained in further detail below, to adjust to the new diameter of the changing body vessel. This maintains the seal between the body region 18 and an interior surface of the body vessel. In addition, it accommodates for the increase in blood flow in vessels due to a patient's growth.

The tubular graft material 14 can comprise a suitable biocompatible material. For example, the tubular graft material 14 may be made of an expanded polytetrafluoroethylene (ePTFE), polytetrafluoroethylene, silicone, polyurethane, polyamide (nylon), as well as other flexible biocompatible materials. The graft bodies also can be made of known fabric graft materials such as woven polyester, polyetherurethanes, or polyethylene, or may comprise a knitted or braided structure or a combination of those. The graft bodies also may include a bioremodelable material such as reconstituted or naturally-derived collagenous materials, extracellular matrix (ECM) material, submucosa, renal capsule membrane, dermal collagen, dura mater, pericardium, fascia lata, serosa, peritoneum or basement membrane layers, or intestinal submucosa, including small intestinal submucosa (SIS), stomach submucosa, urinary bladder submucosa, uterine submucosa, or other suitable materials.

In the present example, the one or more stents 12 generally comprise a zig-zag shape formed, for example, using a wire comprising a plurality of substantially straight segments having a plurality of bent segments disposed therebetween. It will be appreciated that while the stents 12 shown comprise zig-zag configurations, the stents may alternatively or additionally comprise any number of shapes. For example, the stents 12 may comprise a pattern of interconnected struts, including diamond or other shapes as generally known in the art. The stents may be made from a woven wire structure, a laser-cut cannula, individual interconnected rings, or any other type of stent structure that is known in the art.

Moreover, the one or more stents 12 may be made from various metals and alloys. The stents 12 may be made from other metals and alloys that are biased, such that they may be restrained by a delivery device prior to deployment, but are inclined to return to their relaxed, expanded configuration upon deployment. In a preferred embodiment, the stents 12 comprises a self-expanding nitinol or stainless steel stent. In other embodiments, the stents 12 may comprise other materials such as cobalt-chrome alloys, amorphous metals, tantalum, platinum, gold and titanium. The stents 12 also may be made from non-metallic materials, such as thermoplastics and other polymers.

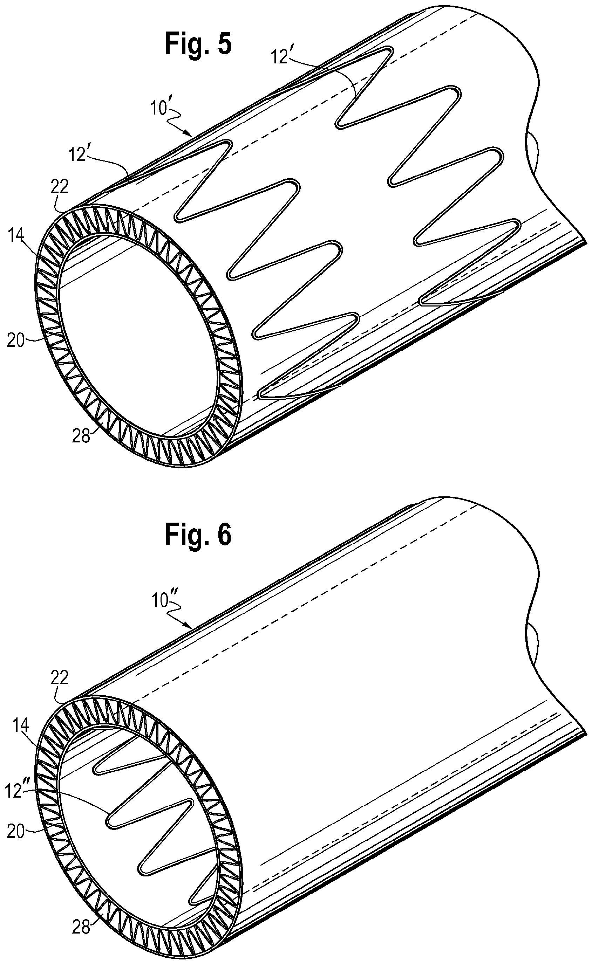

Referring to FIG. 2, the components of the graft 14 of FIG. 1 are shown placed in-between the biodegradable inner layer 20 and the biodegradable outer layer 22. As noted above, the stent 12 is omitted from FIG. 3 for illustrative purposes only, but would be disposed between the outer portion of the graft material 14 and the interior of the outer layer 22 in this example. This is one of at least four possibilities for arrangement of components. As a second possibility, the arrangement of components may be similar to FIGS. 1-2, with the exception that the stent 12 is disposed between the inner portion of the graft material 14 and the outer portion of the inner layer 20. In a third example, the stent may be disposed external to the outer layer 22 as depicted in FIG. 5 and explained further below. In a fourth example, the stent may be disposed internal to the inner layer 20 as depicted in FIG. 6 and explained further below.

The inner and outer layers 20, 22 may comprise multiple layers or may have spatial variations within individual layers. The layers may comprise compositional variations circumferentially with respect to the graft 14 to control biodegradation rate by controlling the graft expansion radially. The layers with different compositional variation may be arranged in segmental form in the longitudinal direction to allow varying radial expansion in different sections of the body region 18 longitudinally. The layers may include biocompatible agents or a coating to minimize or prevent tissue in-growth while maintaining biodegradable properties.

The inner and outer layers 20, 22 may be made from a biodegradable, dissolvable or absorbable material that is substantially impermeable to acids, fluid and the like. By way of example and without limitation, the inner and outer layers 20, 22 may comprise polyactide, polyglycolide, polycaprolactone, polyhydroxylalkanoates, and plyanhydrides or a combination of bioresorbable polymers. For purposes of the present disclosure, such materials are generally referred to collectively as biodegradable, although the mechanisms by which they are degraded, dissolved, absorbed, eroded or otherwise degrade may be different.

As best seen in FIGS. 2-3, the inner and outer layers 20, 22 may be coupled to a portion of the pleats 16 of the graft material 14 using at least one connector 24. In one embodiment, the connectors 24 may comprise a resorbable suture. In other embodiments, the inner and outer layers 20, 22 may be coupled to the graft material at the connectors 24 using a biodegradable adhesive, spraying or dipping process. The connectors 24 can be of multiple types (such as, for example, weld points, stitches, adhesive, and the like). In further embodiments, the connectors 24 may comprise a non-biodegradable material.

In yet another embodiment, the connectors 24 may be formed integrally with the graft material 14. For example, if the graft material 14 is knitted, then the connectors 24 may be formed as part of the yarn material during a weft or warp knitting process, for example, by providing a loop of material extending radially outward from integral courses of the graft material. An integral connector may similarly be formed during a weaving or braiding process.

In some embodiments, the connectors 24 may be omitted. For example, the one or more layers 22 and 24 and the graft material 14 may be created during a single process as a common structure, such as during weaving where the one or more biodegradable layers are woven integrally with the graft fabric 14. In such example, the pleats 16 may be positioned perpendicular to the machine direction. The graft material may be subsequently sewn together to create a tubular structure. In such process, or similar integral processes, it is contemplated that the connectors 24 may be omitted.

As depicted in FIG. 3, in some embodiments tissue growth inhibitors 28 may be embedded within the spaces between the graft material 14. Advantageously, the tissue ingrowth inhibitors 28 may reduce the ability of components of the stent-graft 10 from becoming secured to an inner wall of a vessel or covered with fibrous tissue, which could preclude the ability of the stent-graft to properly self-expand to adapt to the vessel over time, as explained in more detail further below.

The segments of the inner layer 20 and the outer layer 22 that are coupled to the graft material 14 can be designed such that corresponding, adjacent or opposing segments of the inner and outer layers 20 and 22 are degraded at the same, or a substantially similar, rate to allow for uncoupling and subsequent unfolding of the pleats 16 of the graft material 14, as explained further below.

Referring to FIG. 3, several zones or regions of the stent-graft 10 are numbered to help better illustrate certain principles of the disclosure. By way of non-limiting example, selected inner "zones" are numbered X₁ through X₄, selected outer zones are numbered Y₁ through Y5, and selected graft zones are numbered Z₁ and Z₂. It will be appreciated that such labeling is for illustrative purposes only, and that the precise locations of the illustrated zones may differ from what is shown, e.g., to be larger or smaller, or extend further proximally or distally, and the like.

As depicted in FIG. 3, in one example, a first pair of segments X₂ and X₃ in the inner layer 20 and the adjacent or opposing segment Y₃ in the outer layer 22 may be designed to biodegrade at the same or a similar rate, which results in an uncoupling of the graft material 14 between the X₂, X₃ and Y₃ segments. Since the inner layer 20 and the outer layer 22 no longer restrain the pleats 16 of the graft material at this stage, unfolding of the corresponding segments Z₁ and Z₂ of the graft material 14 may be achieved. At this time, the segments Z₁ and Z₂ of the graft material 14 would be able to further expand radially outward, in particular due to the self-expanding nature of the adjacent stents 12 that are coupled to the graft material, and the fact that regions of the inner layer 20 and outer layer 22 have degraded in specific locations and are no longer constraining the stent 12 and graft material 14 near its segments Z₁ and Z₂.

At this stage, the pleats 16 of the graft material 14 would be allowed to unfold to the extent of slack permitted. This would result in an overall increase in the diameter of the stent-graft 10 corresponding to a length of the graft segments Z₁ + Z₂ that were permitted to expand due to degradation of neighboring inner and outer layers 20, 22, coupled with self-expansion of the stent 12 due to the slack permitted. This principle is explained further with respect to FIGS. 4A-4D below.

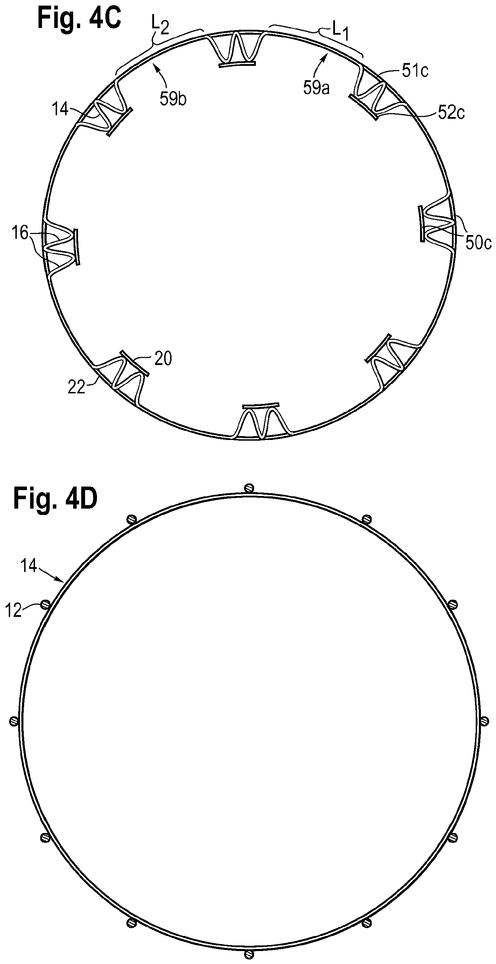

Referring to FIGS. 4A-4D, in one example, additional segment pairs in the inner and outer layers 20 and 22 can be biodegraded substantially simultaneously resulting in the release of multiple folds of the stent-graft 10, depending on the required increase in the stent-graft 10 diameter at each stage (as noted above, the stent 12 is omitted in FIGS. 4A-4C for illustrative purposes only, but its placement is depicted in the final state of FIG. 4D). In this example, around the circumference of the stent-graft 10, there are several different zones, including a first series of zones 50a, a second series of zones 50b, and a third series of zones 50c. Four discrete zones 50a are depicted in cross-hatch lines in FIG. 4A, each comprising an outer layer segment 51a and an inner layer segment 52a. Similarly, four discrete zones 50b are depicted in solid lines in FIG. 4A, each comprising an outer layer segment 51b and an inner layer segment 52b. Finally, the remainder of the stent-graft 10 comprises zones 50c, depicted in clear lines, where each zone 50c comprises an outer layer segment 51c and an inner layer segment 52c.

In FIGS. 4A-4D, it has been selected that the first series of zones 50a will have a first biodegradation rate, which is faster than a second biodegradation rate of the second series of zones 50b. Further, it has been selected that the third series of zones 50c will have a third biodegradation rate, which is slower than the second biodegradation rate of the second series of zones 50b.

FIG. 4A shows the initial folded stent-graft 10 in an implantation or delivery state between the biodegradable inner and outer layers 20 and 22 inside a vessel. In FIG. 4B, after a first predetermined period of time, degradation of the four discrete zones 50a is depicted. In particular, at this stage, the outer layer segments 51a and the inner layer segments 52a of each of the four discrete zones 50a each biodegrade at the first biodegradation rate, which is the fastest. On a more microscopic level, the degradation of each discrete zone 50a may be similar to degradation of the first pair of segments X₂ and X₃ in the inner layer 20 and the opposing segment Y₃ in the outer layer 22, as explained above with respect to FIG. 3.

As the outer layer segments 51a and the inner layer segments 52a of each of the four discrete zones 50a biodegrade, the pleats 16 of the graft material 14 in these areas begin to unfold, assisted by the ability of the stent 12 to self-expand somewhat further. The result is that pleats 16 in each of the four discrete zones 50a become relatively flat and achieve an unfolded length of **L₁** about the circumference of the stent-graft 10, as shown in FIG. 4B. The stent-graft is therefore in a second configuration, having an outer diameter that is larger than the delivery or implantation state of FIG. 4A.

Referring to FIG. 4C, after a second predetermined period of time, which is longer than the first predetermined period of time, degradation of the four discrete zones 50b is depicted. In particular, at this stage, the outer layer segments 51b and the inner layer segments 52b of each of the four discrete zones 50b each biodegrade at the second biodegradation rate, which is slower than the first biodegradation rate of the zones 50a. As the outer layer segments 51b and the inner layer segments 52b of each of the four discrete zones 50b biodegrade, the pleats 16 of the graft material 14 in these areas begin to unfold, assisted by the ability of the stent 12 to self-expand somewhat further. The result is that pleats 16 in each of the four discrete zones 50b become relatively flat and achieve an unfolded length of **L₂** about the circumference of the stent-graft 10, as shown in FIG. 4C. The stent-graft is therefore in a third configuration, having an outer diameter that is larger than the delivery state of FIG. 4A and the first expanded state of FIG. 4B.

It should be noted that while FIGS. 4B-4C depict that the graft 14 unfolds radially outward relative to the remaining constrained pleats 16 (i.e., such that the unfolded graft appears closer to a vessel wall), it is contemplated that the graft 14 may be designed to unfold radially inward relative to the remaining constrained pleats 16 (i.e., such that the unfolded graft appears closer to a lumen of a vessel). Such differences in radial unfolding of the graft 14 may be achieved by selecting specific inner or outer segments to biodegrade.

Referring to FIG. 4D, after a third predetermined period of time, which is longer than the second predetermined period of time, degradation of the remaining zones 50c is depicted. In particular, at this stage, the outer layer segments 51c and the inner layer segments 52c of each of the remaining zones 50b biodegrade at another biodegradation rate, which is slower than the first and second biodegradation rates of the zones 50a and 50b, respectively. As the outer layer segments 51c and the inner layer segments 52c of each of the remaining zones 50c biodegrade, the pleats 16 of the graft material 14 in these areas begin to unfold, assisted by the ability of the stent 12 to self-expand somewhat further. The result is that pleats 16 in each of the remaining zones 50c become relatively flat the stent-graft achieves a final expanded configuration, having an outer diameter that is larger than each of the states in FIGS. 4A-4C. At this time, the graft 14 may be relatively taut such that no further radial expansion can be achieved, even if the stent 12 has an ability to further expand.

Advantageously, in this manner, selective staged expansion of the stent-graft as a whole may be achieved, as generally depicted in FIGS. 4A-4D. In this manner, further invasive surgeries, such as those involving a follow-up procedure to balloon-expand the stent-graft at a later time, may be reduced or avoided altogether.

The stent-graft 10 may advantageously be particularly useful in younger patients, whose vessels may grow over time. Advantageously, the stent-graft 10 can adapt to the growing vasculature while reducing or avoiding follow-up surgeries as the patient gets older.

Notably, a physician may be able to predict vessel expansion of a patient over time, and therefore may select a corresponding stent-graft that may self-expand at the predicted rate. For example, if a patient is approximately 10 years old, and a physician believes an expansion rate of 1-2mm per year of the vessel diameter may occur, then the physician may select a particular stent-graft 10, having specific biodegradation rates of the inner and outer layers 20 and 22, that will match the predicted 1-2mm per year growth of the vessel. Such prediction may be extrapolated until the patient reaches an age, such as 18-20, where little or no further vessel expansion is expected. Therefore, in this non-limiting example, the physician may select a stent-graft 10 having specifically-selected degradation rates to allow the expansion from about age 10 to about ages 18-20, at which point there are no additional pleats 16 to unfold, and thus the final diameter of the stent-graft 10 occurs. In one example, the starting outer diameter of the stent-graft 10 may be in a range of about 5mm-15mm. In one example, the thickness of the inner and outer layers 20, 22 may be at least 10 microns. The degree of biodegradability can be defined by design including varying thickness of layers, material composition of layers, construction of layers and composition of embedded material.

The dimensions of the pleats 16 of the graft material 14 may be selected so that the pleats 16 provide sufficient excess material to allow the graft material to expand radially outward over the required period of time, thus ensuring a sufficient graft material diameter can be achieved as the stent 12 is permitted to self-expand.

A physician may wish to err on the side of predicting relatively slow expansion of the vessel, compared to expansion of the stent-graft 10. This is because it may be preferable if the stent-graft 10 becomes slightly oversized within the vessel, since it will maintain engagement with the intimal surface. On the other hand, if the stent-graft 10 was not expanding at a sufficient rate compared to the vessel, then the stent-graft 10 may lose its engagement with the vessel wall.

While pediatric patients are one particular population that may benefit from the stent-graft 10, it will be appreciated that the stent-graft 10 may be used in other populations, e.g., older patients where a physician may predict vessel expansion over time.

Further, it should be noted that while the stent-graft 10 has been generally referenced for placement in a vessel during an endovascular procedure, it may be used in ducts, passageways or other locations, and may be used in open procedures as well, while achieving similar benefits over time.

It should be noted that during the graft expansion process above, the connectors 24 may stay intact and eventually biodegrade if they comprise degradable material, or may remain permanently in a vessel if they comprise non-biodegradable material.

While the present embodiments provide a highly advantageous design that may reduce further or avoid further invasive surgeries, it should be noted that a physician still has an option to perform a procedure to induce expansion of the stent-graft 10, if needed. For example, if the one or more stents 12 are not self-expanding on their own accord for any reason, or the vessel is expanding unexpectedly fast, then a physician may wish to perform a balloon expansion procedure to induce immediate expansion of the stent-graft 10. In some cases, the stent-graft 10 may expand over time by a combination of the self-expanding abilities noted in FIGS. 4A-4D above, together with periodic surgical procedures to adjust the device is needed.

FIG. 5 is a partial perspective view of another stent-graft 10'. In the embodiment of FIG. 5, the stent-graft 10' is similar to the stent-graft 10 of FIGS. 1-4, with a main exception that the one or more stents 12' are alternatively placed around an exterior surface of the outer layer 22. In this embodiment, the one or more stents 12' may be secured to the graft material 14 using a connector that extends through small holes formed in the outer layer 22. As with the embodiment of FIGS. 1-4, while segment of the inner and outer layers 20 and 22 are degraded, then corresponding regions of the stent-graft 10' are allowed to self-expand over time.

FIG. 6 is a partial perspective view of another stent-graft 10". In the embodiment of FIG. 6, the stent-graft 10" is similar to the stent-grafts 10 and 10' of FIGS. 1-5, with a main exception that the one or more stents 12" are alternatively placed around an interior surface of the inner layer 20. In this embodiment, the one or more stents 12' may be secured to the graft material 14 using a connector that extends through small holes formed in the inner layer 20. As with the embodiments of FIGS. 1-5, while segment of the inner and outer layers 20 and 22 are degraded, then corresponding regions of the stent-graft 10" are allowed to self-expand over time.

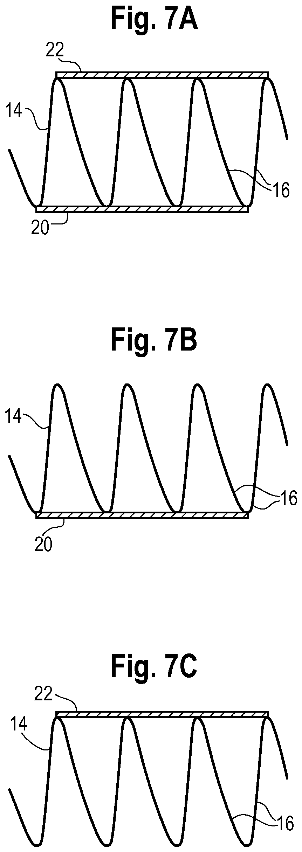

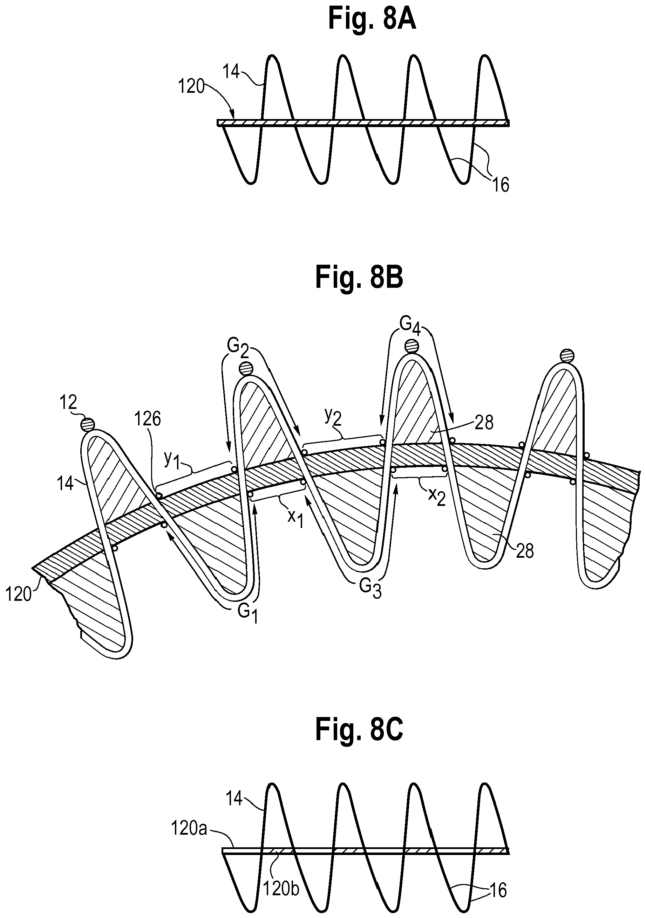

Referring now to FIGS. 7-9, various embodiments are shown explaining how a segment of a graft material may be disposed adjacent to one or more biodegradable layers. It should be noted that, with the exception of FIG. 8B, the stent 12 of FIG. 1 is omitted in FIGS. 7-9 for illustrative purposes, but would be present to help assist with graft expansion during degradation of the one or more biodegradable layers, according to the principles explained in detail above. Moreover, the connectors 24 described above are also omitted from FIGS. 7-9 for illustrative purposes (except for attachment points 126 in FIG. 8B, which may comprise connectors 24), but may be provided according to the description of the connectors 24 in FIG. 3.

Referring to FIG. 7A, an arrangement consistent with FIG. 3 is shown, whereby the graft material 14 is disposed between an inner biodegradable layer 20 and an outer biodegradable layer 22. In FIG. 7B, the graft material 14 is disposed adjacent to an inner biodegradable layer 20, i.e., no outer biodegradable layer is provided. In FIG. 7C, the graft material 14 is disposed adjacent to an outer biodegradable layer 22, i.e., no inner biodegradable layer is provided. In such embodiments where only one biodegradable layer is provided, the pleats 16 of the graft material 14 that are further from the biodegradable layer may be loose in certain locations or may be held in place by an external member, such as the one or more stents 12.

Referring to FIGS. 8A-8C, in other embodiments, a biodegradable layer 120 may extend through the graft material 14, resulting in a series of pleats above and below the biodegradable layer 120. In FIGS. 8A-8B, exemplary biodegradable segments X₁ and X₂ are formed on an inwardly-facing side of the layer 120, and exemplary biodegradable segments Y₁ and Y₂ are formed on an outwardly-facing side of the layer 120. Various folded lengths of graft material G₁ through G₄ are formed surrounding the different biodegradable segments, as depicted in FIG. 8B.

In the embodiment of FIGS. 8A-8B, attachment points 126 are provided for securing the biodegradable layer 120 to the graft material 14. The attachment points 126 may be similar to or comprise the connectors 24 described above. While the attachment points 126 are depicted as external elements, in other embodiments they may be integral with the graft material 14, for examples, formed as integral yarns extending outward from the graft material during the knitting process.

In the example of FIGS. 8A-8B, biodegradation of segment X₁ may cause graft material length G₂ to unfold. Subsequent degradation of segment X₂ may cause graft material length G₄ to unfold. Further degradation of segments may occur to achieve further unfolding of additional graft material portions. It should be noted that while the stent 12 is depicted on an outer portion of the graft material 14 in FIG. 8B, in other embodiments the stent 12 may be disposed internal to the graft material 14.

FIG. 8C is similar to FIGS. 8A-8B, with a main exception that the biodegradable layer 120 comprises multiple segments 120a and 120b, each having a different material. In this embodiment, the biodegradable layer 120 appears as a generally single circumferential layer, however a first material of segment 120a may degrade at a rate different than a second material of segment 120b, thereby allowing differing segments of the graft material 14 to release at different times and locations.

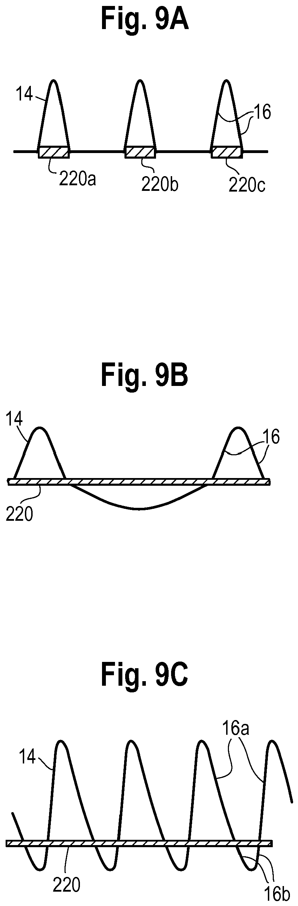

FIG. 9A is similar to FIG. 8C, with a main exception that multiple discrete biodegradable layer segments 220a, 220b, and 220c are provided in a generally circumferentially-aligned manner (i.e., in the same circumferential plane, such that one layer is not significantly further radially inward or outward relative to the other). In this embodiment, the first segment 220a may degrade at a first rate, the second segment 220b may degrade at a second rate, and the third segment 220c may degrade at a third rate, or they may all degrade at the same time.

Referring to FIGS. 9B-9C, further embodiments of a segment of a graft material 14 disposed adjacent to a biodegradable layer are provided. In FIG. 9B, a segment of the graft material 14 is disposed adjacent to a biodegradable layer 220 in an asymmetric or non-sinusoidal manner, such that the pleats 16 of the graft material that are disposed radially outward relative to the layer 220 form a smaller radius of curvature relative to the graft material disposed radially inward relative to the layer 220. In FIG. 9C, the biodegradable layer 220 is disposed radially inward such that pleats 16a comprise a longer length external to the biodegradable layer 220 and other pleats 16b comprise a shorter length internal to the biodegradable layer.

While various embodiments of the invention have been described, the invention is not to be restricted except in light of the attached claims and their equivalents. Moreover, the advantages described herein are not necessarily the only advantages of embodiments of the invention and it is not necessarily expected that every embodiment of the invention will achieve all of the advantages described.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosures in United States patent application number 62/504,212, from which this application claims priority, and in the abstract accompanying this application, are incorporated herein by reference.

## Claims

1. A prosthesis comprising:
a generally tubular graft material having an inner and outer perimeter, and a plurality of pleats between the inner and outer perimeters in a first state;
at least one stent coupled to the graft material; and
at least one layer of biocompatible material coupled to the graft material in the first state,
wherein one or more sections of the at least one layer biodegrade over a first period of time at a first biodegradable rate,
wherein at least one of the plurality of pleats of the graft material unfolds to permit radial expansion of the graft material after the first period of time, wherein the stent provides support for the radial expansion of the graft material.

2. The prosthesis of claim 1, wherein after a final period of time longer than the first period of time, the at least one layer is substantially entirely biodegraded to define a maximally expanded state.

3. The prosthesis of claim 1 or 2, wherein the at least one layer comprises an inner layer of biodegradable material disposed on the inner surface of the graft material, and further comprises at least one outer layer of biodegradable material disposed on the outer surface of the graft material.

4. The prosthesis of claim 3, wherein a segment of the inner layer and an adjacent segment of the outer layer comprise the same biodegradation rate.

5. The prosthesis of claims 1 or 2, where a single layer of biodegradable material is provided and disposed through a central region of the graft material.

6. The prosthesis of any preceding claim, where biodegradable material in a first series of zones comprises the first biodegradation rate, which is faster than a second biodegradation rate of biodegradable material in a second series of zones.

7. The prosthesis of claim 6, where the first series of zones are discrete and spaced-apart from the second series of zones by a third series of zones.

8. The prosthesis of claim 7, where biodegradable material in the third series of zones comprises a third biodegradation rate, which is slower than the first and second biodegradation rates.

9. The prosthesis of any preceding claim, wherein the pleats are substantially parallel to a longitudinal axis of the prosthesis.

10. The prosthesis of any preceding claim, wherein the at least one layer comprises biocompatible agents or tissue growth inhibitors.

11. A prosthesis comprising:
a generally tubular graft material having an inner and outer perimeter, and a plurality of pleats between the inner and outer perimeters in a first state;
at least one stent coupled to the graft material;
at least one inner layer of biodegradable material disposed on the inner surface of the graft material in the first state;
at least one outer layer of biodegradable material disposed on the outer surface of the graft material in the first state,
wherein a first section of the inner layer biodegrades over a first period of time at a first biodegradable rate that corresponds to a period of time in which a second section of the outer layer biodegrades, wherein the first section of the inner layer is disposed adjacent to the second section of the outer layer,
wherein at least one of the plurality of pleats of the graft material unfolds to permit radial expansion of the graft material after the first period of time, wherein the stent provides support for the radial expansion of the graft material.

12. The prosthesis of claim 11, wherein the pleats are substantially parallel to a longitudinal axis of the prosthesis.

13. The prosthesis of claim 11 or 12, wherein a segment of the inner layer, and an adjacent segment of the outer layer, comprise the same biodegradation rate.

14. The prosthesis of any of claims 11 to 13, where biodegradable material in a first series of zones comprises the first biodegradation rate, which is faster than a second biodegradation rate of biodegradable material in a second series of zones.

15. The prosthesis of claim 14, where the first series of zones are discrete and spaced-apart from the second series of zones by a third series of zones.
